# DEMANDE DE BREVET EUROPEEN

(11) **EP 1 064 932 A1**
(43) Date de publication de la demande: **03.01.2001**
(21) Numéro de dépôt: 00401621.8
(22) Date de dépôt: 08.06.2000
(51) Int. Cl.: A61K 7/48, A61K 7/32

(54) **Utilisation de cellules végétales dédifférenciées**

(30) Priorité: 02.07.1999 FR 9908569
(71) Demandeur: L'OREAL, 75008 Paris (FR)
(72) Inventeur: Hilaire, Pascal, 37210 Vouvray (FR); Martin, Richard, 37210 Rochecorbon (FR); Courbiere, Christophe, 75015 Paris (FR)
(74) Mandataire: Galup, Cédric Olivier Nicolas

(57) **Abrégé**

L'invention se rapporte à l'utilisation d'au moins un extrait de cellules végétales dédifférenciées à l'exception des cellules du genre Bellis, dans ou pour la préparation d'une composition, l'extrait ou la composition étant destinés à inhiber les odeurs.

L'invention a en outre pour objet une composition d'hygiène corporelle et/ou déodorante ainsi qu'un procédé de traitement cosmétique destiné à inhiber les odeurs.

## Description

L'invention se rapporte à l'utilisation d'au moins un extrait de cellules végétales dédifférenciées à l'exception des cellules du genre Bellis, dans ou pour la préparation d'une composition, l'extrait ou la composition étant destinés à inhiber les odeurs.
L'invention a en outre pour objet une composition d'hygiène corporelle et/ou déodorante ainsi qu'un procédé de traitement cosmétique destiné à inhiber les odeurs.

L'anatomie et la physiologie de la peau varient d'une partie à l'autre du corps. Mais quelle que soit la région anatomique, la peau contient des glandes sébacées et sudoripares dont les excrétions sont constituées entre autres d'eau, d'acides aminés, d'urée, d'électrolytes et/ou d'acides gras spécifiques. Outre que ces excrétions représentent un excellent milieu nutritif pour l'ensemble de la flore, principalement bactérienne, qui colonise la peau, leurs composants une fois au contact de l'air subissent des réactions chimiques comme par exemple l'oxydation, qui les dégradent et donnent naissances à des produits responsables d'odeurs corporelles qui peuvent parfois s'avérer gênantes.
Certaines substances inhibitrices naturelles (bactéricides et/ou bactériostatiques) dues à la dégradation partielle des lipides complexes sécrétés par les glandes sudoripares sont volatils et peuvent être associées à une forte odeur que l'on a coutume de combattre.

Cependant, les excrétions cutanées ne sont pas les seules responsables des odeurs corporelles. La flore cutanée elle-même en est en partie responsable.

Dans le domaine cosmétique, il est bien connu d'utiliser en application topique, des produits déodorants contenant des substances actives de type antitranspirant ou de type bactéricide pour diminuer voire supprimer les odeurs corporelles généralement désagréables.

Les substances antitranspirantes ont pour effet de limiter le flux sudoral. Elles sont généralement constituées de sels d'aluminium qui d'une part sont irritants pour la peau et qui d'autre part diminuent le flux sudoral en modifiant la physiologie cutanée, ce qui n'est pas satisfaisant.
Les substances bactéricides, en inhibant le développement de la flore cutanée responsable des odeurs corporelles, présentent l'inconvénient de ne pas être actifs sur l'odeur de la sueur déjà développée. Ces produits bactéricides, dont le plus couramment employé est le triclosan (5-chloro-2-(2,4-dichlorophénoxy) phénol), sont donc à long terme insuffisamment efficaces.
De plus, les déodorants peuvent modifier la microflore, principalement vers les bactéries Gram-négatives, et déclencher en conséquence des infections.

Il demeure donc intéressant de pouvoir disposer dans le cadre du traitement des odeurs corporelles de composés et/ou de compositions efficaces et ne présentant pas d'effets secondaires.

A cet égard la demanderesse à maintenant découvert de manière surprenante et inattendue que des extraits de cellules végétales dédifférenciées peuvent inhiber les odeurs, particulièrement les odeurs liées à la sueur humaine.

L'invention a donc pour objet premier l'utilisation dans une composition ou pour la préparation d'une composition d'au moins un extrait de cellules végétales dédifférenciées, à l'exception des cellules du genre Bellis, l'extrait ou la composition étant destinés à inhiber les odeurs.

Particulièrement selon l'invention on utilise au moins un extrait de cellules végétales dédifférenciées pour inhiber les odeurs corporelles et encore plus particulièrement les odeurs dues à la sueur animale ou humaine.
Préférentiellement l'invention s'adresse à la sueur humaine.

Par cellules végétales dédifférenciées, on entend toute cellule végétale ne présentant aucun des caractères d'une spécialisation particulière et capable de vivre par elle-même et non en dépendance avec d'autres cellules.

Les cellules végétales dédifférenciées peuvent être obtenues à partir de matériel végétal issu de plante entière ou de partie de plante comme les feuilles, les tiges, les fleurs, les pétales, les racines, les fruits, les graines, les anthères.

Préférentiellement, les cellules végétales dédifférenciées sont obtenues à partir de feuilles.

Les cellules végétales dédifférenciées utilisables selon l'invention peuvent être obtenues à partir de végétaux obtenus par culture *in vivo* ou issu de culture *in vitro.*

Par culture *in vivo* on entend toute culture de type classique c'est à dire en sol à l'air libre ou en serre ou encore hors sol.

Par culture *in vitro,* on entend l'ensemble des techniques connues de l'homme du métier qui permet de manière artificielle l'obtention d'un végétal ou d'une partie d'un végétal. La pression de sélection imposée par les conditions physico-chimiques lors de la croissance des cellules végétales *in vitro* permet d'obtenir un matériel végétal standardisé et disponible tout au long de l'année contrairement aux plantes cultivées *in vivo.*

Préférentiellement selon l'invention on utilise des cellules végétales dédifférenciées issues de culture *in vitro.*

Les cellules végétales dédifférenciées utilisables selon l'invention peuvent être obtenues par toute méthode connue de l'art antérieur. A cet égard on peut citer les méthodes décrites par E.F. George et P.D. Sherrington dans Plant Propagation by tissue culture, handbook and directory of commercial laboratories (Exegetics Ltd 1984).
Les milieux de culture utilisables selon l'invention sont ceux généralement connus de l'homme du métier. On peut citer à titre d'exemples les milieux de Gamborg, Murashige et Skoog, Heller, White etc.... On trouvera dans "Plant Culture Média : formulations and uses" de E.F. George, DJM Puttock et H.J George (Exegetics Ltd 1987, tome 1 & 2) des descriptions complètes de ces milieux.

Préférentiellement selon l'invention on prépare les cellules végétales dédifférenciées cultivées sur milieu de Murashige et Skoog.

Toute méthode d'extraction connue de l'homme du métier peut être utilisée pour préparer l'extrait selon l'invention.

Ainsi, les extraits utilisables selon l'invention peuvent prendre toute forme connue. On peut, en particulier, citer les extraits aqueux, alcooliques, notamment éthanoliques ou encore hydroalcooliques.
Préférentiellement selon l'invention, l'extrait est un extrait aqueux.

On peut également utiliser un extrait préparé par la méthode décrite dans la demande de brevet français n° 95-02379 déposée par la demanderesse.

Ainsi, dans une première étape on broie le matériel végétal dans une solution aqueuse à froid et dans une deuxième étape les particules en suspension sont éliminées de la solution aqueuse issue de la première étape. Cette solution aqueuse correspond à l'extrait.
Eventuellement, dans une troisième étape on stérilise la solution aqueuse issue de la deuxième étape.

Avantageusement, l'extrait peut dans une étape subséquente être lyophilisé.

La première étape peut être avantageusement remplacée par une opération de congélation simple des tissus végétaux (par exemple à -20°C ou encore à -180°C dans l'azote liquide), suivie d'une extraction aqueuse reprenant les deuxième et troisième étapes ci-dessus décrites.

Le traitement à froid permet de geler les activités enzymatiques, la filtration stérilisante évite la dégradation des actifs par les micro-organismes de l'environnement. Enfin, le véhicule eau est compatible avec les récepteurs ex vivo et facilite les formulations cosmétiques.

Il est connu que les extraits végétaux contiennent, outre des protéases qui peuvent nuire à la qualité de l'extrait, des oxydases responsables, entre autres, de l'oxydation desdits extraits. Or une telle oxydation conduit à une coloration marron foncée des extraits et à une odeur âcre rendant ceux-ci peu compatibles avec leur utilisation en cosmétique. Dans cet ordre d'idée on connaît en particulier une laccase dont le poids moléculaire est supérieur à 100000 daltons.

Ainsi, avantageusement, l'extrait obtenu peut être fractionné par toute méthode de fractionnement connue permettant d'éliminer les oxydases et en particulier la polyphénoloxydase. On peut par exemple filtrer l'extrait de l'invention sur une membrane de dialyse afin d'en éliminer les molécules d'un poids moléculaire supérieur à 100000 daltons. Il est également possible de faire subir à l'extrait un fractionnement par précipitations sélectives.

D'autres méthodes permettent de se prémunir des phénomènes d'oxydation. En particulier, l'extrait peut également être stabilisé. Toute méthode de stabilisation connue peut être utilisée selon l'invention. On peut par exemple stabiliser l'extrait de l'invention par barbotage d'azote pour éliminer l'oxygène dissout ou encore en y ajoutant de la cystéine et/ ou des dérivés soufrés à une concentration finale comprise entre 0,5 g/l et 10 g/l et de préférence entre 1 g/l et 2,5 g/l.

Bien évidemment l'extrait selon l'invention peut être fractionné et stabilisé.

L'extrait peut constituer à lui seul le principe actif des compositions de l'invention.

Un exemple de préparation d'extrait utilisable selon l'invention est donné par ailleurs dans les exemples.

La quantité d'extrait utilisable selon l'invention est bien entendu fonction de l'effet recherché et peut donc varier dans une large mesure.

Pour donner un ordre de grandeur, on peut utiliser un extrait tel que défini précédemment en une quantité représentant de 0,01% à 20% du poids total de la composition et préférentiellement en une quantité représentant de 0,1% à 5% du poids total de la composition.

Les cellules végétales dédifférenciées utilisables selon l'invention peuvent provenir de toutes les espèces de végétaux connues.
A cet égard on citera les genres Salvia, Coleus, Rosmarinus, Ginkgo, Cannabis, Colchicum, Gloriosa, Asparagus, Glycine, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum et Iris.

Particulièrement selon l'invention on utilise des cellules végétales dédifférenciées provenant de végétaux des genres Ginkgo, Theobroma, Salvia ou Datura.

Bien entendu les extraits de cellules végétales dédifférenciées utilisables selon l'invention peuvent provenir de mélanges de cellules végétales dédifférenciées obtenues à partir de genres végétaux différents et/ou obtenues à partir de matériel végétal différent.

L'invention a comme second objet une composition d'hygiène corporelle, caractérisée par le fait qu'elle comprend dans un milieu physiologiquement acceptable au moins un extrait de cellules végétales dédifférenciées, à l'exception des cellules du genre Bellis. La composition d'hygiène corporelle peut être une composition déodorante.

La composition de l'invention peut être une composition cosmétique ou dermatologique. Préférentiellement selon l'invention, la composition est une composition cosmétique, particulièrement d'application topique.

La présente invention a en outre pour objet un procédé de traitement cosmétique de la peau destiné à inhiber les odeurs, caractérisé par le fait que l'on applique sur la peau, sur les cheveux, et/ou sur les muqueuses, une composition cosmétique comprenant au moins un extrait de cellules végétales dédifférenciées à l'exception des cellules du genre Bellis.

Le procédé de traitement cosmétique de l'invention peut être mis en oeuvre notamment en appliquant les compositions cosmétiques telles que définies ci-dessus, selon la technique d'utilisation habituelle de ces compositions. Par exemple : application de crèmes, de gels, de sérums, de lotions, de laits, de shampooings ou de compositions anti-solaires sur la peau.

Les exemples et compositions suivants illustrent l'invention sans la limiter aucunement. Dans les compositions les proportions indiquées sont des pourcentages en poids.

### Exemple 1 : Préparation de cellules végétales dédifférenciées de Salvia miltiorrhiza (sauge) :

Après prélèvement de feuilles, celui-ci est décontaminé avec des solutions saturées d'hypochlorite de sodium ou de calcium à une concentration de 290 g/l, à température ambiante (25°C) pendant un temps compris entre 1 à 5 minutes. Les tissus sont rincés à l'eau distillée stérile puis placés dans une solution d'éthanol dilué. Ils subissent 3 lavages à l'eau distillée stérile en fin de décontamination.
Les explants sont alors découpés stérilement sous hotte à flux laminaire puis placés en boite de Pétri sur milieu Murashige et Skoog. Les cals primaires apparaissent entre 2 et 5 semaines. Les cals primaires sont alors repiqués sur une gélose neuve préparée avec le même milieu de culture. Au fur et à mesure des repiquages, les cellules se stabilisent (aspect, couleur ...) et sont transférables en milieu liquide pour être éventuellement cultivées de façon industrielle en fermenteur.

| milieu Murashige et Skoog : | |
|---|---|
| Macroéléments de Skoog | 100,00 ml |
| Microéléments de Skoog | 1,00 ml |
| Vitamines de Skoog | 2,00 . ml |
| Fer/EDTA | 10,00 ml |
| acide 2-4 Dichlorophenoxyacetique 10⁻⁴M | 10,00 ml |
| Kinétine 10⁻⁴ | 0,60 ml |
| Saccharose | 30,00 g |
| Agar | 8,00 g |
| Eau distillée | qsp 1 Litre |
| pH avant stérilisation | 5,8 UpH |

Stérilisation 115 ou 121°C durant 20 à 40 minutes

| Macro éléments en mg/l de Skoog | |
|---|---|
| KNO₃ | 1900 |
| NH₄NO₃ | 1650 |
| MgSO₄, 7 H₂0 | 370 |
| CaCl₂, 2 H₂0 | 440 |
| KH₂PO₄ | 170 |

| Micro éléments en mg/l de Skoog | |
|---|---|
| CuSO₄, 5 H₂0 | 0,025 |
| MnSO₄, 1 H₂0 | 16,90 |
| Kl | 0,83 |
| Na₂MoO₄, 2 H₂0 | 0,25 |
| ZnSO₄, 7 H₂0 | 10,60 |
| H₃BO₃ | 6,20 |
| CoCl₂, 6 H₂0 | 0.025 |

| Vitamines mg/l de Skoog | |
|---|---|
| Myoinositol | 100,00 |
| Acide nicotinique | 0,50 |
| Pyridoxine | 0,50 |
| Thiamine | 0,10 |
| Fer/EDTA | |
| FeSO₄, 7 H₂0 | 27.8 |
| Na₂ EDTA | 37.3 |

### Exemple 2 : Préparation des extraits :

10 grammes de cellules stabilisées obtenues à l'exemple 1, sont récupérées par filtration sur toile de 50 à 100 µm suivant la taille des agrégats cellulaires. Les cellules sont placées à -20°C en congélation lente propice à la formation de cristaux intracellulaires de grande taille permettant de casser les cellules par cryobroyage (congélateur).
Les cellules décongelées sont placées à 4°C puis broyées plus finement au Potter dans 10 ml d'eau distillée. 2 passages au broyeur sont ainsi effectués. Les matières en suspension sont éliminées par centrifugation à 8000 G, pendant 10 minutes à 4°C.
On effectue une pré-filtration du surnageant sur papier Whatman GF/F de 7 cm de diamètre puis une filtration à 0,22µm pour éliminer les particules fines restant en suspension.
On obtient environ 12 ml d'extrait directement utilisables
Les produits ainsi obtenus sont lyophilisés. Après lyophilisation, on obtient environ 1 gramme d'extrait (poids sec).

On place 0,1 grammes de chaque extrait dans un pilulier contenant 1 ml de sueur fraîche.
La sueur fraîche est récupérée grâce à un vêtement étanche placé sur chaque donneur de sueur avant mise en sauna.

### Exemple 3 : évaluation olfactive de l'effet inhibiteur d'extraits de cellules végétales dédifférenciées préparés selon l'exemple 1

L'évaluation est réalisée par quatre testeurs entraînés.
L'évaluation de l'odeur de chaque échantillon est réalisée de façon aléatoire et notée.
Les deux critères d'évaluation ainsi que les notations sont les suivants:
- Intensité globale :

| Note | 0 | 1 | 2 | 3 | 4 |
|---|---|---|---|---|---|
| Intensité | Nulle | Légère | Modérée | Forte | Très forte |

- Appréciation hédonique:

| Note | |
|---|---|
| 0 | Très agréable |
| 1 | Agréable |
| 2 | Légèrement agréable |
| 3 | Neutre |
| 4 | Légèrement désagréable |
| 5 | Désagréable |
| 6 | Très désagréable |

Une seconde évaluation peut être réalisée 6 heures après la première.

### Matériel et méthode :

Un pool de sueur axillaire humaine mélangée provenant de 4 à 6 sujets est produit par stimulation thermique (sauna à environ 80°C et 20-30% d'humidité relative pendant 2 heures). La sueur fraîche est récupérée grâce à un vêtement étanche placé sur chaque donneur de sueur avant mise en sauna.

100 mg d'extrait sont introduits dans un pilulier de 30 ml en verre blanc (⌀ de l'ouverture 25 mm) avec un bouchon protégé intérieurement par une feuille d'aluminium.
1 ml de sueur fraîche est introduit dans chaque flacon.
L'ensemble est agité modérément.
Le pH est évalué à l'aide de bandelettes réactives.
Les flacons sont bouchés puis incubés à 37 °C.
L'évaluation olfactive est réalisée après 18 heures d'incubation.
L'intensité et le désagrément d'odeur sont positionnés par rapport à un échantillon de sueur sans ajout d'actif parfaitement identifié et qui est évalué en premier et réévalué autant de fois que nécessaire. Un témoin d'efficacité non identifié contenant 0.8 mg de triclosan (agent bactériostatique) ainsi qu'un témoin sueur sans ajout d'actif mais non identifié sont rajoutés dans la série d'échantillon à évaluer.

### Evaluation :

Les flacons sont sortis après 18 heures d'incubation en étuve thermostatée à 37°C. Ils sont ouverts et placés sous hotte ventilée 10 minutes minimum avant de les évaluer.

### Résultats :

Les moyennes des notes attribuées par chacun des évaluateurs est calculée pour chaque extrait. Les résultats sont exprimés en pourcentage de variation d'intensité et de note hédonique par rapport au témoin sans actif. Plus les pourcentages de variation obtenus sont élevés, plus l'efficacité de l'extrait est importante.

| Genre | % inhibition intensité | % Variation hédonique |
|---|---|---|
| Ginkgo cultivé à la lumière | 23,1 | 28,3 |
| Ginkgo cultivé à l'obscurité | 23,1 | 37,0 |
| Salvia cultivé à l'obscurité | 26,9 | 14,0 |
| Cacao cultivé à l'obscurité | 37,9 | 15,6 |
| Datura cultivé à la lumière | 20,7 | 13,3 |

### Exemple 4 : Exemples de compositions selon l'invention. Ces compositions sont obtenues par les techniques habituelles couramment utilisées en cosmétique.

| Composition 1 : lotion déodorante | |
|---|---|
| Palmitate de 2-éthyl hexyle | 35,00 g |
| Cyclopenta dimethylsiloxane | 6,60 g |
| Butylène glycol | 5,00 g |
| Extrait de sauge . | 2,50 g |
| Conservateur | qs |
| Eau déminéralisée | qsp 100,00 g |

| Composition 2 : crème déodorante | |
|---|---|
| Mélange d'alcool cétylstéarylique / alcool cétylstéarylique 30 OE | 7,00 g |
| Mono / distéarate de glycérol | 2,00 g |
| Huile de vaseline | 15,00 g |
| Glycérine | 20,00 g |
| Extrait de sauge | 5,00 g |
| Parfum, conservateur | qs |
| Eau déminéralisée | qsp 100,00 g |

| Composition 3 : lait déodorant | |
|---|---|
| Palmitate de 2-éthyl hexyle | 35,00 g |
| Glycérine | 2,00 g |
| Extrait de sauge | 3,00 g |
| Copolymère acide acrylique / acrylate d'alkyle C10/C30 réticulé | 0,10 g |
| Triethanolamine | 0,10 g |
| Acides aminés de blé en solution aqueuse | 1,00 g |
| Conservateurs qs | |
| Eau déminéralisée qsp | 100,00 g |

| Composition 4 : gel déodorant | |
|---|---|
| Behenoyl lactylate de sodium | 10,00 g |
| Glycérine | 2,00 g |
| Extrait de sauge | 2,50 g |
| Ricinoléate de zinc | 1,00 g |
| Acide stéarique | 8,40 g |
| Hydroxyde de sodium | 1,20 g |
| Parfum, colorants, conservateurs | qs |
| Eau déminéralisée | qsp 100,00 g |

| Composition 5 : émulsion eau dans silicones | |
|---|---|
| Silicone DC 245 Fluid (DOW CORNING) | 6,60 g |
| Silicone DC 5225 C (DOW CORNING) | 9,40 g |
| Alcool éthylique | 11,00 g |
| Extrait de sauge | 3,00 g |
| Propylène glycol | 37,00 g |
| Parfum, conservateurs | qs |
| Eau déminéralisée | qsp 100,00 g |

| Composition 6 : Stick déodorant alcoolique | |
|---|---|
| Acide stéarique | 7,00 g |
| Hydroxyde de sodium | 1,08 g |
| Myristate d'isopropyle | 5,00 g |
| Extrait de sauge | 2,50 g |
| Alcool éthylique | 60,80 g |
| Propylene glycol | 20,50 g |
| Parfum qs | |
| Eau déminéralisée | qsp. 100,00 g |

| Composition 7 : Stick antitranspirant anhydre | |
|---|---|
| Alcool stéarylique | 22.00 g |
| Huile de ricin hydrogénée | 5,00 g |
| Palmitate d'isopropyle | 12,50 g |
| Hexachlorure d'Aluminium anhydre | 20,00 g |
| Extrait de sauge | 0,50 g |
| Cyclopenta diméthylsioxane | 35,00 g |
| Talc | 5,00 g |

## Revendications

1. Utilisation dans une composition ou pour la préparation d'une composition d'au moins un extrait de cellules végétales dédifférenciées, à l'exception des cellules du genre Bellis, l'extrait ou la composition étant destinés à inhiber les odeurs.

2. Utilisation selon la revendication 1, caractérisée par le fait que les odeurs sont des odeurs corporelles.

3. Utilisation selon l'une quelconque des revendications 1 ou 2, caractérisée par le fait que les odeurs sont dues à la sueur animale ou humaine.

4. Utilisation selon l'une quelconque des revendications 1 à 3, caractérisée par le fait que les odeurs sont dues à la sueur humaine.

5. Utilisation selon l'une quelconque des revendications 1 à 4, caractérisée par le fait que les cellules végétales dédifférenciées sont obtenues à partir de matériel végétal issu de plante entière ou de partie de plante.

6. Utilisation selon la revendication 5, caractérisée par le fait que les cellules végétales dédifférenciées sont obtenues à partir de matériel végétal issu de plante entière ou de partie de plante comme les feuilles, les tiges, les fleurs, les pétales, les racines, les fruits, les graines, les anthères.

7. Utilisation selon l'une quelconque des revendications 5 ou 6, caractérisée par le fait que les cellules végétales dédifférenciées sont obtenues à partir de feuilles.

8. Utilisation selon l'une quelconque des revendications 1 à 7, caractérisée par le fait que les cellules végétales dédifférenciées sont obtenues par culture *in vivo* ou issu de culture *in vitro.*

9. Utilisation selon la revendication 8, caractérisée par le fait que les cellules végétales dédifférenciées sont obtenues par culture *in vitro.*

10. Utilisation selon l'une quelconque des revendications 1 à 9, caractérisée par le fait que l'extrait est un extrait aqueux, alcooliques ou hydroalcooliques.

11. Utilisation selon la revendication 10, caractérisée par le fait que l'extrait est un extrait aqueux.

12. Utilisation selon l'une quelconque des revendications 1 à 11, caractérisée par le fait que l'extrait est en une quantité représentant de 0,01% à 20% du poids total de la composition.

13. Utilisation selon la revendication 12, caractérisée par le fait que l'extrait est en une quantité représentant de 0,1% à 5% du poids total de la composition.

14. Utilisation selon l'une quelconque des revendications 1 à 13, caractérisée par le fait que les cellules végétales dédifférenciées proviennent de végétaux des genres Salvia, Coleus, Rosmarinus, Ginkgo, Cannabis, Colchicum, Gloriosa, Asparagus, Glycine, Medicago, Mungo, Erythrina, Oenothera, Papaver, Atropa, Datura, Solanum, Borago, Reseda, Amsonia, Catharantus, Pilocarpus, Digitalis, Coffea, Theobroma, Jasminum, Capsicum et Iris.

15. Utilisation selon la revendication 14, caractérisée par le fait que les cellules végétales dédifférenciées proviennent de végétaux des genres Ginkgo, Theobroma, Salvia ou Datura.

16. Composition d'hygiène corporelle, caractérisée par le fait qu'elle comprend dans un milieu physiologiquement acceptable au moins un extrait de cellules végétales dédifférenciées telles que décrites dans l'une quelconques des revendications 1 à 15.

17. Composition déodorante, caractérisée par le fait qu'elle comprend dans un milieu physiologiquement acceptable au moins un extrait de cellules végétales dédifférenciées telles que décrites dans l'une quelconque des revendications 1 à 15.

18. Composition selon l'une quelconque des revendications 16 ou 17, caractérisée par le fait qu'elle est d'application topique.

19. Procédé de traitement cosmétique de la peau destiné à inhiber les odeurs, caractérisé par le fait que l'on applique sur la peau, sur les cheveux, et/ou sur les muqueuses, une composition cosmétique telle que décrite dans l'une quelconque des revendications 16 à 18.
